# EUROPEAN PATENT APPLICATION

(11) **EP 1 074 259 A1**
(43) Date of publication of application: **07.02.2001**
(21) Application number: 99910696.6
(22) Date of filing: 25.03.1999
(51) Int. Cl.: A61K 31/70, A61K 31/425, A61K 45/00

(54) **PREVENTIVES FOR ALCOHOLIC GASTRITIS**

(30) Priority: 01.04.1998 JP 8867298
(71) Applicant: CHUGAI SEIYAKU KABUSHIKI KAISHA, Tokyo, 115-8543 (JP)
(72) Inventor: IGUSA, Kazuo, Tokyo 192-0913 (JP); YAMAZAKI, Tamotsu, Chugai Seiyaku Kabushiki Kaisha, Toshima-ku, Tokyo 171-8545 (JP)
(74) Representative: VOSSIUS & PARTNER
(86) International application number: JP9901509
(87) International publication number: WO9949872

(57) **Abstract**

A prophylactic drug for the prevention of acute alcoholic gastritis, comprising a combination of sucralfate and a histamine H₂-receptor antagonist, particularly preferably famotidine, is disclosed. This prophylactic drug shows a marked synergistic action in preventing acute alcoholic gastritis, in comparison with each of the constituent drugs being used alone. The combined use of the two drugs enables a marked reduction in the respective necessary doses.

## Description

### TECHNICAL FIELD

This invention relates to a novel prophylactic drug aimed at the prevention of alcoholic gastritis. More specifically, the invention relates to a prophylactic drug which has as its object the prevention of alcoholic gastritis, which comprises a combination of sucralfate and a histamine H₂-receptor antagonist, and preferably a combination of sucralfate and famotidine.

### BACKGROUND ART

Illness following excessive drinking is a state most adults have experienced. The main cause of illness following excessive drinking is considered to be due to a buildup of acetaldehyde, the primary metabolite of alcohol.

A hangover can be characterized as a state of fatigue in which acute gastritis, in vivo electrolyte imbalance, and dehydration, all contribute to a state of illness. Obviously, moderate ingestion of alcohol is the most effective way of preventing a hangover. It is also well known that the ingestion of honey, a substance rich in fructose which assists in the process of oxidation of alcohol in vivo, vitamin preparations, and the ingestion of foods rich in vitamins such as liver, yeast, fresh vegetables and lemon can also be of benefit.

Acute gastritis due to gastric mucosal irritation by alcohol gives rise to various symptoms ranging from abdominal discomfort to pain, which may also be accompanied by nausea and vomiting, and fever. In the presence of such abdominal discomfort or pain, histopathological examination shows mucosal edema, desquamation of the epithelium, clear cellular infiltration, erosion and shallow ulceration. Irritation due to hypersecretion of gastric juice is also present, aggravating discomfort.

To deal with such symptoms, it is generally necessary to take a gastrointestinal drug available at drug stores and containing a mucosa repairing agent, an antacid, and stomachic pharmacognosical products for, for example, 2 to 3 days. During this period, anorexia, malaise, and weakness often persist, and conventional gastrointestinal drugs have been unsuccessful in producing a satisfactory therapeutic effect in alleviating the symptoms of hangover.

### DISCLOSURE OF THE INVENTION

The inventors of the present invention have conducted extensive studies in an attempt to resolve gastric disturbance associated with drinking, and to develop a highly safe drug for the treatment of alcoholic gastritis. As a result they have found that a prophylactic drug for the prevention of alcoholic gastritis which is excellent in efficacy and safety can be obtained by combining a histamine H₂-receptor antagonist, such as cimetidine, ranitidine, nizatidine, roxatidine, or famotidine, and sucralfate, which is a drug used in the treatment of gastrointestinal disease. This finding has led to the accomplishment of the present invention.

That is, the invention relates to a prophylactic drug for the prevention of alcoholic gastritis, comprising a combination of sucralfate and a histamine H₂-receptor antagonist.

### BRIEF DESCRIPTION OF THE DRAWING

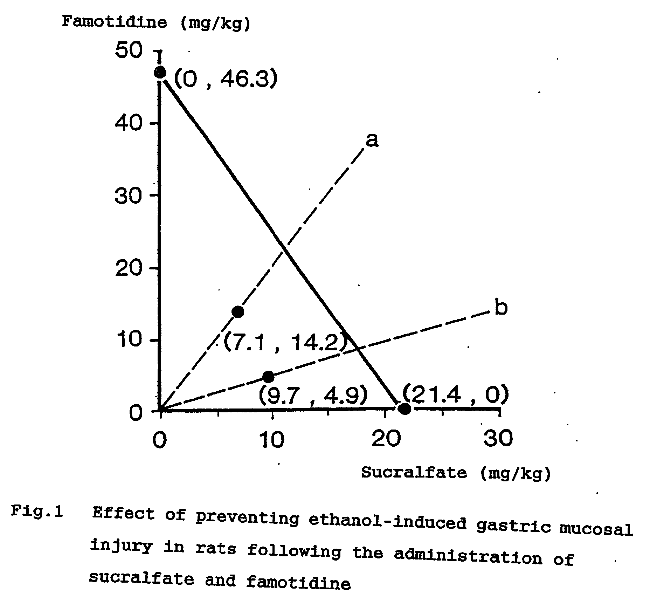

FIG. 1 is a graphic representation of an equivalent curve showing the ED₅₀ values for the effect of preventing ethanol-induced gastric mucosal injury in rats following the administration of sucralfate and famotidine at a ratio of 1:2 (a) or 2:1 (b).

### EMBODIMENTS OF THE INVENTION

The histamine H₂-receptor antagonist as used in the invention is a drug which exhibits a gastric acid secretion suppressing action by antagonizing the histamine H₂-receptor of the gastric mucosal parietal cell. By this mechanism, this drug proves useful in the treatment of gastric ulcer, duodenal ulcer, reflux esophagitis, and gastritis. As the histamine H₂-receptor antagonist of the invention, cimetidine, ranitidine, nizatidine, roxatidine, or famotidine is cited.

The histamine H₂-receptor antagonist will be described in further detail, with famotidine being taken as an example, but the invention is not restricted to famotidine.

In the invention, a combination of famotidine and sucralfate has been found to produce a marked synergistic action in preventing alcoholic gastritis. Refer, for example, to a study of the preventive effect of this combination on alcoholic gastritis in rat models of gastritis induced by 99.5% ethanol, as shown in Examples to provided later. The study involving two combinations of sucralfate and famotidine in a ratio of 2:1 or 1:2 demonstrated a marked synergistic action for ED₅₀ (the dose for preventing alcoholic gastritis in 50% of the animal subjects), as shown in FIG. 1. Particularly regarding ED₅₀ values at a sucralfate/famotidine ratio of 2:1, a comparable pharmacodynamic effect was obtained at a famotidine dose of about 1/10 of the dose of famotidine administered alone, and at a sucralfate dose of about 1/2 of the dose of sucralfate administered alone. This combination can also be expected to produce a marked antagonistic action in terms of safety.

The marked synergistic effect of the above sucralfate-famotidine combination has been unknown hitherto, and has been made available for the first time by the present invention. The prophylactic drug of this invention is excellent both in terms of efficacy and safety, which factors determine the value of a medicine. Accordingly, this drug is highly valuable.

The proportion of sucralfate to famotidine is preferably 1 part (part by weight; the same applies to the description to follow) of the former to 0.05 to 10 parts, more preferably, 0.1 to 5 parts, of the latter. When the proportion of sucralfate to famotidine is outside this range, the above-mentioned synergistic effect is not achieved markedly.

To the prophylactic drug of the invention, auxiliary drugs, such as antacids, stomachics, digestives, drugs for controlling intestinal function, analgesic antispasmodics, and other mucosa repairing agents, may be further added.

The prophylactic drug of the invention can be used in various dosage forms such as tablets, granules, powders, capsules and liquids.

The present invention will now be described in greater detail based on the following Examples, which in no way limit the invention.

### [Examples]

Rats were forced to abstain from food and water for 24 hours, and used as experimental subjects. Sucralfate and famotidine were used as test drugs, and the empirical ED₅₀ of each drug was set as the treatment dose. When the two drugs were combined, the dose of the combination treatment was set such that the proportion of sucralfate was 1/3 of the dose, and the proportion of famotidine was 2/3 of the dose, or vice versa (i.e., the proportion of sucralfate was 2/3 of the dose, and the proportion of famotidine was 1/3 of the dose).

For each of the individual treatments and the combination treatments, a high dose group, a medium dose group, and a low dose group were provided by setting a high dose, a medium dose, and a low dose at a ratio of 9:3:1. Each dose level of the drug was suspended in a 2% aqueous solution of acacia. The suspension was orally administered in a volume of 2 ml/kg body weight, while only the solvent was administered in the same volume for a control group. A total of 13 groups, consisting of 3-dose-level groups for two individual-treatment modes and two combination-treatment modes, and one control group, with 10 animals included in each group, were compared under the same conditions to determine ED₅₀ values. One hour after administration of the test drug, 99.5% ethanol in an amount of 1 ml/animal was administered orally.

One hour later, the animal subjects were sacrificed and the stomach removed, treated, and measured for the extent of injuries occurring in the gastric mucosa. The total amount of injuries occurring In the respective animals was calculated to determine the ED₅₀ values for the preventive effect of the test drug on gastric mucosal injury. The results are shown in Table 1 and FIG. 1. These results proved that the combination of the two drugs exhibited a synergistic effect; namely, these drugs, when combined, prevented injury to the gastric mucosa at low doses.

**Table 1**

| n = 10/group | | |
|---|---|---|
| Sucralfate:Famotidine (mg/kg, P.O.) | Mucosal injury suppression rate (%) | 50% preventive dose ED₅₀ (mg/kg) |
| 0:0 | 0 | - |
| 10:0 | 19 | |
| 30:0 | 69 | Sucralfate 21.4 |
| 90:0 | 92 | |
| 6.6:3.3 | 36 | Sucralfate 9.7 |
| 20:10 | 76 | |
| 60:30 | 81 | Famotidine 4.9 |
| 3.3:6.6 | 24 | Sucralfate 7.1 |
| 10:20 | 69 | |
| 30:60 | 84 | Famotidine 14.2 |
| 0:10 | 13 | |
| 0:30 | 42 | Famotidine 46.3 |
| 0:90 | 66 | |

### INDUSTRIAL APPLICABILITY

When taken about 1 hour before drinking, the prophylactic composition as a drug according to the present invention can suppress alcohol-induced injury to the gastric mucosa following the ingestion of alcohol, and can alleviate the symptoms of subsequent acute gastritis, such as abdominal discomfort and pain.

## Claims

1. A prophylactic drug for the prevention of alcoholic gastritis, comprising a combination of sucralfate and a histamine H₂-receptor antagonist.

2. A prophylactic drug for the prevention of alcoholic gastritis as claimed in claim 1, wherein the histamine H₂-receptor antagonist is famotidine.

3. The prophylactic drug of claim 2, wherein the histamine H₂-receptor antagonist is contained in an amount of 0.1 to 5 parts by weight per part by weight of sucralfate.

4. The prophylactic drug of claim 1, 2 or 3, wherein an antacid, a stomachic, a digestive, a drug for controlling intestinal function, an analgesic antispasmodic, and other mucosa repairing agent are further contained.
